(19) Europäisches Patentamt
European Patent Office
Office européen des brevets

(11) EP 2 062 600 A1

(12) EUROPEAN PATENT APPLICATION

(43) Date of publication:
**27.05.2009 Bulletin 2009/22**

(51) Int Cl.:
***A61K 49/18*** *(2006.01)*

(21) Application number: **07120180.0**

(22) Date of filing: **07.11.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Koninklijke Philips Electronics N.V.
5621 BA Eindhoven (NL)**

(72) Inventor: **The designation of the inventor has not yet been filed**

(74) Representative: **Van Velzen, Maaike Mathilde
Philips
Intellectual Property & Standards
P.O. Box 220
5600 AE Eindhoven (NL)**

(54) **CEST MRI contrast agents based on non-spherical particles**

(57) In magnetic resonance imaging (MRI) based on chemical exchange-dependent saturation transfer (CEST), a carrier for CEST contrast agents is provided which comprises a paramagnetic chemical shift reagent and a source of protons with which the chemical shift reagent can interact, so as to provide chemically shifted protons. It is desired for a CEST contrast agent to be non-spherical, so as to allow a bulk magnetic susceptibility effect of the pool of protons within the agent to contribute to the CEST effect. Intrinsically non-spherical carriers are provided on the basis of erythrocyte ghosts.

EP 2 062 600 A1

Printed by Jouve, 75001 PARIS (FR)

## Description

FIELD OF THE INVENTION

The invention relates to Magnetic Resonance Imaging (MRI) based on Chemical Exchange-dependent Saturation Transfer (CEST). More particularly, the invention relates to CEST MRI contrast agents based on non-spherical carriers comprising a lipid bilayer shell enclosing a paramagnetic chemical shift reagent and a pool of protons.

BACKGROUND OF THE INVENTION

Magnetic Resonance Imaging (MRI) is an important diagnostic technique that is commonly used in hospitals for the diagnosis of disease. MRI allows for the non-invasive imaging of soft tissue with a superb spatial resolution.

Almost all current MRI scans are based on the imaging of bulk water molecules, which are present at a very high concentration throughout the whole body in all tissues. If the contrast between different tissues is insufficient to obtain clinical information, MRI contrast agents (CAs), such as low molecular weight complexes of gadolinium, are administered. These paramagnetic complexes reduce the longitudinal (T1) and transverse relaxation times (T2) of the protons of water molecules. Drawbacks of these T1-based contrast agents are their relatively low contrast efficiency especially at high magnetic field strengths (B0 larger than 1.5 T) and their rapid renal excretion.

An alternative method to generate image contrast utilizes Chemical Exchange-dependent Saturation Transfer (CEST) from selected, magnetically pre-saturated protons to the bulk water molecules. CEST in combination with a paramagnetic chemical shift reagent (ParaCEST) is a promising new MRI method. In this technique, the magnetization of a pool of paramagnetically shifted protons of a CEST contrast agent is selectively saturated by the application of radio frequency (RF) radiation. The transfer of this saturation to bulk water molecules by proton exchange leads to a reduced amount of excitable water protons in the environment of the CEST contrast agent. Thus a decrease of the bulk water signal intensity is observed, which can be used to create a (negative) contrast enhancement in MRI images.

An approach to obtain a high CEST efficiency is based on utilizing the large number of water molecules of a solution containing a paramagnetic shift reagent (e.g. Na[Tm(dotma)($H_2O$)]), wherein"$H_4$dotma" stands for α,α',α,''α'''-tetramethyl-1,4,7,19-tetraacetic acid and dotma represents the respective fourfold deprotonated tetraanionic form of the ligand, to provide a pool of protons that are chemically shifted and that, therefore, can selectively be saturated by an RF pulse. If this system is encapsulated in a carrier, e.g. a liposome, the magnetic saturation can be transferred to the bulk water molecules, at the outside of the carriers, which are not chemically shifted (LipoCEST). The amount of magnetization transfer and hence the extent of contrast enhancement are determined by the rate of the diffusion of water through the shell of the carrier, e.g. a phospholipid membrane, as well as by the amount of water within the carrier.

The optimum water exchange rate is directly correlated with the chemical shift difference between the proton pool inside of the carrier and the bulk water outside of the carrier. The paramagnetic shift that is induced on the water molecules inside the liposomes consists of two main contributions: chemical shift resulting from a direct dipolar interaction between the water molecules and the shift reagent ($\delta_{dip}$), and chemical shift caused by a bulk magnetic susceptibility effect ($\delta_{bms}$). The overall paramagnetic shift is the sum of these two contributions:

$$\delta = \delta_{dip} + \delta_{bms} \qquad (1)$$

$\delta_{bms}$ is zero for spherical particles, but it can be significant for anisotropic particles. The aspherical particles experience a force in a magnetic field, which causes them to align with the magnetic field lines. In the case of liposomes, this effect is further increased, if they bear paramagnetic material associated with the phospholipid membrane.

A reference on CEST using aspherical liposomes is Terreno,E. et al. Angew. Chem. Int. Ed. 46, 966-968 (2007).

The practical, and notably *in vivo* applicability of CEST agents based on aspherical liposomes is prone to drawbacks. In general, liposomes are intrinsically spherical. To deform them, they are subjected to a dialysis process against a hypertonic buffer solution, hence a buffer solution with a higher osmolarity compared to the solution at the inside of the liposomes. The dialysis causes a net diffusion of water from the inside of the liposomes to the bulk solution. This reduces the total inner volume of the liposomes. Since the surface area of the liposomes remains constant, the volume reduction forces the liposomes to deform and to assume an aspherical shape, such as a disk shape, a cigar shape, or any other aspherical shape. This deformation is not permanent but reversible, since exposure of these so deformed liposomes to an isotonic or hypotonic buffer solution, results in a recovery of their original spherical shape. Therefore, the extent of the induced paramagnetic shift difference depends on the osmolarity of the surrounding bulk solution. This factor is more difficult to control in *in-vivo* applications, since in this case the bulk buffer solution is replaced with body fluids.

Further, the application of CEST agents based on liposomes *in vivo* is potentially hampered by the rapid clearance of liposomes from the blood by macrophage uptake.

SUMMARY OF THE INVENTION

It would be advantageous to provide aspherical CEST contrast agents that are not prone to becoming spherical. Particularly, it is desired to provide aspherical CEST contrast agents that maintain an aspherical shape also when they are in direct contact with body fluids. Yet a further desire is to provide carriers, of such a type as can be filled with a pool of protons, that are intrinsically aspherical and thus do not require deformation to be optimized for use as a CEST contrast agent. It also were advantageous to provide aspherical CEST contrast agents that are intrinsically biocompatible, and that are preferably based on a natural, particularly human source. A particular desire in this respect is to provide aspherical CEST contrast agents that are applicable in personalized medicine methods, by being based on a material harvested from the person to be subjected to MRI using the CEST contrast agents.

In order to better address the above desires and advantages, in one aspect of the invention, a contrast agent for proton MRI based on CEST is provided, the agent comprising a non-spherical carrier comprising a lipid bilayer shell enclosing a paramagnetic chemical shift reagent that is in contact with a pool of protons, wherein the non-spherical carrier is an erythrocyte ghost.

In another aspect of the invention, a method of performing a CEST MRI scan is provided, wherein contrast agents based on erythrocyte ghosts are introduced into body fluid of a person subjected to the MRI.

In yet another aspect of the invention, the use is presented of erythrocyte ghosts as a carrier for MRI based on Chemical Exchange-dependent Saturation Transfer.

DETAILED DESCRIPTION OF THE INVENTION

In one aspect of the invention, it is found that a paramagnetic chemical shift reagent can be encapsulated in erythrocytes. Since, as a result of the introduction of the paramagnetic chemical shift reagent, the erythrocytes have lost most, and preferably all, of their original water-soluble contents, the resulting, paramagnetic chemical shift reagent-loaded erythrocytes, are more appropriately referred to as erythrocyte ghosts. Thus, carriers result in which a paramagnetic chemical shift reagent is contained in a membrane which happens to be the phospholipid bilayer originating from an erythrocyte.

Erythrocytes are reported to be potential biocompatible vectors for bioactive substances, e.g. drugs. A review on this use of erythrocytes is Millan,C.G., et al., "Drug, enzyme and peptide delivery using erythrocytes as carriers." Journal of Controlled Release 95, 27-49 (2004).

Previously described applications include the encapsulation of MRI $T_1$ and $T_2$ contrast agents. The present invention relates to an entirely different type of MRI contrast enhancement, viz. CEST, and to benefits specific to CEST.

By making use of erythrocyte ghosts, the present invention effectively utilizes the erythrocyte membrane as a shell for a CEST contrast agent.

Erythrocytes that can be utilized in the present invention are preferably erythrocytes originating from species that have erythrocytes without a nucleus. Thus, it is preferred to use mammalian erythrocytes, and most preferably human erythrocytes. The latter provides an additional advantage of inherent biocompatibility. Also, it opens up the possibility of using erythrocytes harvested from the same person as will be subjected to MRI using the resulting, chemical shift-reagent loaded erythrocyte ghosts.

The paramagnetic chemical shift reagent-loaded erythrocyte ghosts are obtainable by a process comprising the steps of providing erythrocytes, subjecting the erythrocyte to hypotonic lysis so as to provide an opening in the erythrocyte membrane, subjecting the opened erythrocyte to one or more washing steps so as to substitute a solution or dispersion of a paramagnetic chemical shift reagent for at least part of the original water-soluble remove contents of the erythrocyte, and subjecting the resulting paramagnetic chemical shift reagent-loaded erythrocyte ghosts to a closing step under isotonic conditions.

Although, preferably, the washing and filing steps are conducted as a concerted action, it is also possible to conduct the washing steps as separate washing and filling steps, whereby in a first step, or first series of steps, the opened erythrocytes are subjected to a washing step so as to remove the original water-soluble contents (preferably all of the cell cytoplasm), and in a second step, or a second series of steps, the thus emptied erythrocytes are contacted with a paramagnetic chemical shift reagent under such conditions as will allow the paramagnetic chemical shift reagent to enter the emptied erythrocytes. E.g. by contacting the emptied erythrocytes with an aqueous solution or dispersion of a paramagnetic chemical shift reagent. Such contacting can be done, e.g. by incubating the opened erythrocytes in an aqueous solution or dispersion of the paramagnetic chemical shift reagent.

Although, in theory, the paramagnetic chemical shift reagent could be introduced from a different solution or dispersion than an aqueous one, it is preferred to first provide an aqueous solution or dispersion of the shift reagent,

and then bringing this solution or dispersion into contact with opened erythrocytes. For, it is required that the resulting paramagnetic chemical shift reagent-loaded erythrocytes comprise said shift reagent in conjunction with a pool of protons. Besides being a new aqueous solution brought into the erythrocytes as explained above, the pool of protons could, at least partially be based on water originally present in the natural erythrocytes. The pool of protons could also be provided by a different type of solution or dispersion of the paramagnetic chemical shift reagent, such as an aqueous buffer solution, a micro- or nanoemulsion of a not-water soluble liquid in water, an aqueous solution of liposomes, or any combination of the above solutions. In particular the solution, emulsion, or suspension comprising the pool of protons may contain further compounds, e.g. polymeric compounds, that may serve to enhance the CEST effect or that may serve a different purpose. By providing, however, the paramagnetic chemical shift reagent as a solution or dispersion in water and introducing this solution or dispersion into the erythrocytes, it is well-controlled that the paramagnetic chemical shift reagent can interact with protons as required, within the resulting loaded erythrocyte ghosts.

The pool of protons is typically water, but can also be formed of other molecules which allow proton exchange through the lipid bilater shell of the erythrocytes in the contrast agent of the invention. Thus, e.g. metabolites may be used to provide at least one of the proton pools. Although the invention is described with reference to proton MRI, it is conceivable that the agent, instead of comprising a pool of protons, comprises a pool of other MRI analytes. In the event of MRI on the basis of nuclei different from protons, the chemical shift reagent in the carrier, and the presence of such nuclei in the environment of the contrast agent when conducting an MRI scan, will have to be adapted accordingly, as will be apparent to the person skilled in the art.

The loaded erythrocyte ghosts have the advantage, as particularly displayed in CEST MRI, of being intrinsically aspherical. The process of loading the erythrocytes with a paramagnetic chemical shift reagent does not change this. It cannot be excluded that the filled erythrocytes of the invention, depending on use or storage, may be prone to changes in shape, but any such changes do not substantially affect the use in CEST MRI, since the erythrocytes are stably aspherical. This stable aspherical shape is mainly caused by the internal cellular skeleton. The aspherical shape contributes to the above-mentioned bulk susceptibility effect $\delta_{bms}$.

Other than with liposomes, which are made aspherical by dialysis against a surrounding hypotonic buffer solution, the aspherical shape of the CEST contrast agents based on erythrocytes is not dependent on the osmolarity of the surrounding bulk solution into which they are brought. This obviates a drawback of the *in vivo* application of liposomes. In the latter case, the substitution of body fluids for the controlled, hypotonic surrounding buffer solution leads to the inherent difficulty to control the environment required to maintain an aspherical shape. The erythrocyte-based CEST contrast agents of the invention desirably are not dependent on the nature of the surrounding solution. Thus, in the case of the contrast agents of the invention, the extent of the induced paramagnetic shift difference is stable irrespective of the surrounding bulk solution.

The paramagnetic chemical shift reagent or reagents can basically be any paramagnetic agent suitable to render the relatively large number of water molecules of a solution or dispersion in which it is contained, into a pool of protons that are chemically shifted regarding their MR resonance frequency, with respect to the surrounding protons of the bulk water molecules. As explained hereinbefore with respect to lipoCEST, the erythrocyte ghosts in which the paramagnetic chemical shift reagent and the internal pool of protons are contained, can be saturated through an RF pulse. As the erythrocytes fundamentally allow exchange of protons with their direct environment, the saturation caused by the RF pulse will be transferred to the environment of the loaded, RF-saturated, erythrocyte ghosts. Thus, upon conducting magnetic resonance imaging, the direct environment of the erythrocyte ghosts will show a decreased signal intensity as compared to other bulk water molecules, and thus allows to detect the direct environment of the contrast agents due to a decreased signal intensity, which results in a negative contrast enhancement.

The paramagnetic chemical shift reagent is to comprise a paramagnetic compound, i.e. any compound having paramagnetic properties. Preferably the paramagnetic compound comprises a paramagnetic metal, where the term metal refers to metallic nano- or microparticles, or metal ions, explicitely including metal ions complexed by chelate ligands. Paramagnetic metals are known to the skilled person, and do not require elucidation here. E.g., early and late transition metals, explicitely including chromium, manganese, iron, as well as lanthanides, such as gadolinium, europium, dysprosium, holmium, erbium, thulium, ytterbium.

The paramagnetic chemical shift reagent is to comprise a chelating structure capable of strongly binding to the paramagnetic metal and allowing the metal to interact with water, or with another suitable source of protons. With respect to suitable chelating structures, reference is made to P. Caravan et al., Chem. Rev., 99, 2293-2352 (1999). Preferably the water is at least transiently coordinated to the metal of the paramagnetic reagent. With respect to chemical shift mechanisms, reference is made to J. A. Peeters et al., Prog. Nucl. Magn. Reson. Spectr., 28, 283-350 (1999). In one embodiment, the chelating structure itself also comprises exchangeable protons, e.g. hydroxy, amine, or amide protons.

Suitably, the paramagnetic chemical shift reagent comprises a lanthanide ion coordinated with a chelating structure, e.g. macrocylic lanthanide(III) chelates derived from 1,4,7,10-tetraazacyclododecane-1,4,7,10-tetraacetic acid ($H_4$DOTA), 1,4,7,10-tetraazacyclododecane-$\alpha$,$\alpha$',$\alpha$,''$\alpha$'''-tetramethyl-1,4,7,10-tetraacetic acid ($H_4$DOTMA), and related ligands that allow for an axially coordinated water molecule in the paramagnetic reagent. In this respect reference is

made to Aime et al., Angew. Chem. Int. Ed., 44, 5513-5515 (2005).

Preferably, the paramagnetic chemical shift reagent is water-soluble. Suitable chemical shift reagents are known to the person skilled in the art. The CEST contrast agents do not require any specific chemical shift reagent, as long as the shift reagent and the pool of protons have a sufficient interaction to result in a pool of chemically shifted protons.

Preferably, the paramagnetic shift reagent is a metal complex comprising a metal ion and a ligand that is based on a multidentate chelate ligand. More preferably, the interaction of the chemical shift reagent with the pool of protons is provided in the form of coordination. Thus it is preferred for the metal complex to have at least one coordination site of the metal left open for the coordination of at least one water molecule.

Examples of suitable water-soluble chemical shift reagents are [Ln(hpdo3a)($H_2O$)] (1), [Ln(dota)($H_2O$)]$^-$ (2), [Ln(dotma)($H_2O$)]$^-$ (3), [Ln(dotam)($H_2O$)]$^{3+}$ (4), and [Ln(dtpa)($H_2O$)]$^{2-}$ (5), including derivatives thereof and related compounds, with Ln being a lanthanide ion.

Preferably the paramagnetic chemical shift reagent is a lanthanide complex such as in formulae 1-5 below:

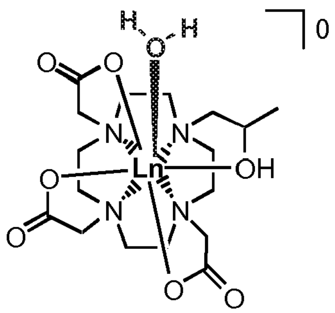

[Ln(hpdo3a)($H_2O$)]

**1**

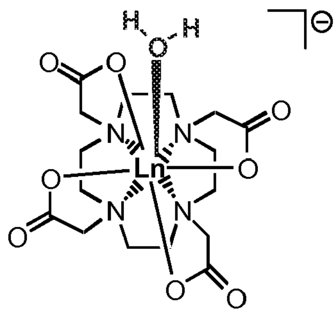

[Ln(dota)($H_2O$)]$^-$

**2**

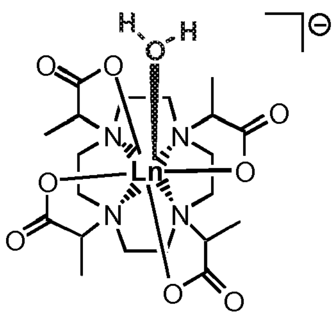

[Ln(dotma)($H_2O$)]$^-$

**3**

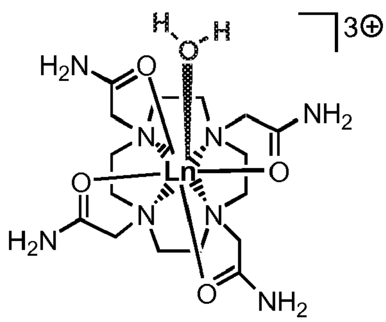

[Ln(dotam)($H_2O$)]$^{3+}$

**4**

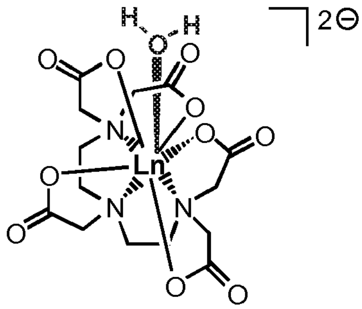

$[Ln(dtpa)(H_2O)]^{2-}$

**5**

wherein the lanthanide is $Eu^{3+}$, $Dy^{3+}$, $Ho^{3+}$, $Er^{3+}$, $Tm^{3+}$, $b^{3+}$, and preferably is $Tm^{3+}$ or $Dy^{3+}$.

The paramagnetic chemical shift reagent is typically comprised in the agent in an amount of from 1 mM to 2000 mM, preferably of from 10 mM to 1000 mM, and more preferably of from 50 mM to 200 mM.

The CEST contrast agents may comprise a $T_1$ or $T_2$ reduction agent. In this respect reference is made to Aime et al., JACS, 129, 2430-2431 (2007). In this way an all-in-one concept is realized of $T_1$, $T_2$, and CEST contrast.

The chemical shift difference to the saturation transfer protons in the environment of the erythrocyte ghosts, can be further enhanced by providing the erythrocyte ghost's membrane with a further paramagnetic agent, which is not necessarily a chemical shift reagent. Thus, the orientation of the aspherical carrier in the magnetic field is affected and the aforementioned bulk susceptibility effect is enhanced. The further paramagnetic agent is preferably an amphiphilic compound comprising a lanthanide complex (on the more polar side of the amphilic compound), and having an apolar tail which has a tendency to preferably integrate in and align with the lipid bilayer at the erythrocyte ghost's surface based on hydrophobic molecular interactions.

These amphilic paramagnetic complexes can e.g. satisfy any of the following formulae 6 to 10:

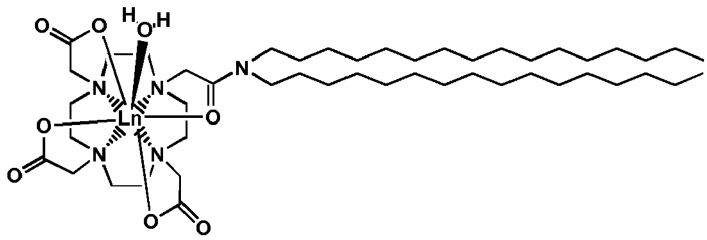

**6**

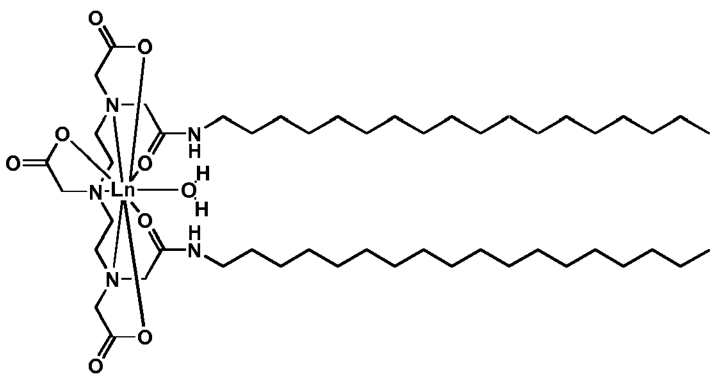

**7**

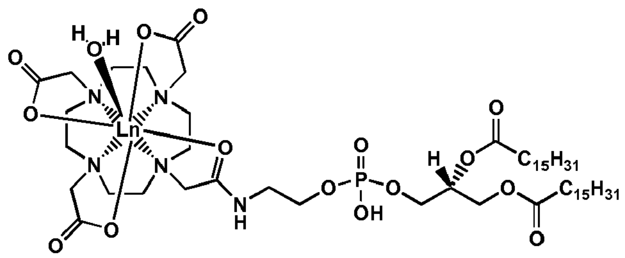

8

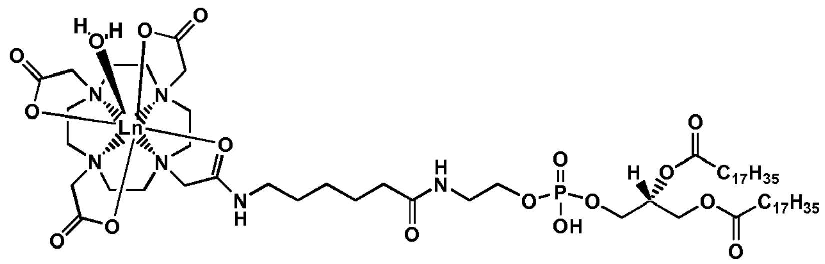

9

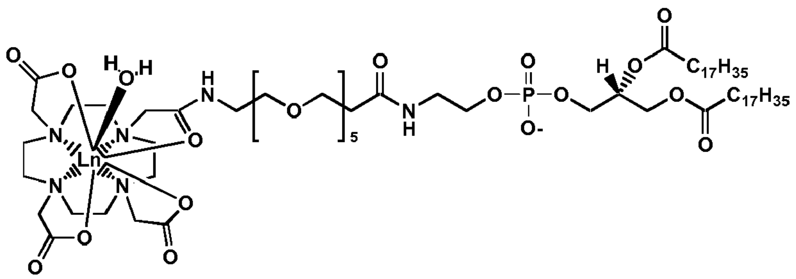

10

The lanthanide ion in the optional membrane-associated paramagnetic agent may be identical with or different from the lanthanide within the cavity of the contrast agent.

As is provided according to the invention, the paramagnetic chemical shift reagent can be encapsulated in erythrocyte ghosts. In this way a pool of water protons is created that has a different chemical shift compared to that of the bulk water surrounding the ghosts. The magnetic resonance of these chemically-shifted water protons can be saturated with an RF pulse of a sufficiently narrow band width. Since the water molecules at the inside of the contrast agent are exchanging quickly with the bulk water molecules surrounding the contrast agents, this saturation is transferred to the bulk water.

Hence, when used in practice, at the location of a CEST contrast agent based on erythrocyte ghosts, the surrounding water (i.e. body fluid in the preferred use *in vivo*) will be visible as hypointense areas in the CEST-enhanced MR images. With CEST-enhanced MRI, we mean conventional MRI wherein, prior to excitation the exchangeable-water resonance has been selectively saturated. The RF pulse used for saturation typically has a band-width of several Hertz to several hundred Hertz. The appropriate frequency for the pulse is usually known a priori from phantom or preclinical CEST-MRI studies, but can also be optimized during the actual clinical MRI examination.

The CEST contrast agents according to the invention can be used in a variety of ways. They can be applied to generate a desired level of MRI contrast in any aqueous environment. Its main use, which is also where the benefits of using erythrocyte ghosts are enjoyed most, is to generate a local MRI contrast upon in vivo application. This can be by introducing the contrast agents, e.g. by injection into the blood or another body fluid of a living being, preferably a human being, and to perform a CEST contrast-enhanced MRI scan of the body, in whole or in part, of said being. The CEST contrast enhancement of bulk water molecules generated, allows the visibility of spots, such as tumors, where the regular body fluid presence is disturbed. Also, the contrast agents of the invention, in their lipid shell can be provided with disease-specific molecular probes, e.g. by having compounds possessing a hydrophobic tail suitable to penetrate into the surface of the carrier (e.g. in the case of a phospholipid surface), wherein the other end of the compounds contains a ligand as desired.

This allows the contrast agents to preferentially locate at desired or suspect body sites which then can be made visible by MRI.

The CEST contrast agents of the invention act on the basis of a pool of protons within the carrier, which exchange with fluid outside of the carrier. This exchange can be done by water-proton transfer, but also by proton transfer from other molecules small enough to pass the lipid bilayer of the erythrocytes.

An additional benefit of the present invention is that it allows a novel aspect of so-called "personalized medicine," which generally indicates diagnostics, treatment and prevention methods specifically tuned to the individual patient. This is carried out in the present invention by harvesting the erythrocyte ghosts to be used as CEST contrast agents from the person to be subjected to the MRI. Apart from the possibility that this reduces the chance of side-effects of the MRI contrast agents, even without a proven effect, it may be perceived as an advantage that persons will appreciate, e.g. based on safety considerations with respect to the reduced risk of inheriting infectious diseases.

The invention also pertains to a method of performing a CEST MRI scan on a person, wherein CEST contrast agents are brought into body fluid of the person and wherein the MRI method includes the application of an RF pulse for which chemically shifted protons in the agent are receptive, so as to saturate the magnetization of said protons, and allowing sufficient time to detect the transfer of said saturation to protons in the outside environment of the contrast agents. Said time typically is of the order of a few seconds.

In one such CEST contrast enhanced MRI scan, typically of from 10-12 M to 10-4 M (1 pM - 0.1 mM) of CEST contrast agents according to the invention are used.

It is to be understood that the invention is not limited to the embodiments and formulae as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

The invention will be illustrated with reference to the following, non-limiting Example and the accompanying non-limiting Figures.

BRIEF DESCRIPTION OF THE DRAWINGS

Fig. 1 shows a method for the preparation of erythrocyte ghosts loaded with a chemical shift reagent for (CEST) MRI applications.

Depicted are an erythrocyte (1) which is subjected to hypotonic lysis (I), so as to form a opened erythrocyte (2). The opened erythrocyte (2) is subjected to a washing step (II), so as to form an erythrocyte ghost from which the original water-soluble contents have been removed (3). The erythrocyte ghost (3) is subjected to an encapsulation and resealing step (III) so as to result in an erythrocyte ghost filled with a solution or dispersion of a paramagnetic chemical shift reagent (4).

Fig. 2, below, shows CEST spectra of erythrocyte ghost loaded with a paramagnetic chemical shift reagent (Na[Tm(dotma)($H_2O$)]) and of erythrocyte ghosts without said reagent as a control experiment.

The squares ( ■ ) indicate the spectrum of the erythrocyte ghosts with Na[Tm(dotma)($H_2O$)]. The dots ( ● ) indicate the spectrum of the control experiment.

On the vertical (Y) axis the CEST effect (in %) is plotted against the saturation offset (in Hz) on the horizontal (X) axis.

EXAMPLE 1

<u>Preparation of a contrast agent of the invention.</u>

CEST contrast agents were prepared from human blood that was collected from a healthy volunteer. Whole blood (about 6 mL) was centrifuged. 3 mL of the cell-containing sample fraction (1, packed cells, about $10^{10}$ cells per mL) were diluted with 27 mL of hypotonic lysis buffer and incubated at 0°C for 30 minutes (Figure 1). Lysed cells were centrifuged for 10 minutes at 30.000 g at 0°C to collect cell membranes (Figure 1, step i). The membrane fraction (2) was washed once with about 27 mL of hypotonic lysis buffer, centrifuged and the supernatant was discarded (Figure 1, step ii). A total of about 3 mL of packed ghost membranes (3) was obtained. The sample was split in two portions, A and B. To 0.8 mL of suspension A were quickly added 0.1 mL of a buffer solution containing 0.6 M Na[Tm(dotma)($H_2O$)], and 0.1 mL of a buffer solution containing 0.8 M NaCl. To 0.8 mL of suspension B were quickly added 0.1 mL of the buffer solution and 0.1 mL of a buffer solution containing 1.4 M NaCl. Subsequently both samples were treated in the same way: samples were incubated at 37°C for 30 minutes to re-seal the membranes to obtain erythrocyte ghosts. Completion of the sealing process was confirmed by determination of the cell volume with a Coulter Counter. The samples were stored at 4°C overnight. To each of the samples (about 1 mL each) was added 9 mL of the buffer solution, they were mixed briefly and centrifuged at 3000 g for 10 minutes at 4°C. The supernatant was removed and the erythrocyte

ghosts were washed an additional 6 times to obtain 1 mL of final ghost suspensions. Both samples were subsequently characterized by (CEST) MR experiments.

EXAMPLE 2

CEST MR spectroscopy

5 mm diameter NMR tubes (Wilmad Labglass) were filled with 450 mL of freshly prepared solutions of ghost cells. To enable frequency locking, a coaxial glass capillary insert (Wilmad Labglass) filled with deuterated tetrachloroethane was used. All MR data were recorded at 310 K on a Bruker Avance NMR spectrometer equipped with an Oxford wide-bore 7 T superconducting magnet. First, a regular one-dimensional pulse-acquire MR spectrum (128k complex data points, 6.2 ms dwell time) was recorded in order to find the resonance frequency of the bulk-water protons. For a CEST-MR spectrum, a series of presaturation-pulse-acquire MR spectra (4k complex data points, 0.2 ms dwell time) were acquired using standard continuous-wave irradiation (2 seconds pulse duration; ca 2 mT pulse amplitude) for selective presaturation of the exchangeable proton resonance. Typically, 121 spectra were acquired at different values of the presaturation offset frequency (100 - 200 Hz intervals) centered around the bulk-water resonance frequency (as obtained from the pulse-acquire experiment) and stored in a single two-dimensional MR data set. To reconstruct the one-dimensional CEST spectrum (Figure 2), the bulk-water signal of each individual spectrum in the two-dimensional data set was integrated. From these bulk-water intensities the CEST effect was calculated according to the following equation and plotted as a function of the presaturation offset frequency.

$$\text{CEST effect} = (1 - MS/M0^*) \times 100\%$$

Here, MS is the bulk-water intensity after an RF saturation pulse applied at a specific offset frequency (e.g. the exchangeable-proton resonance frequency of the contrast agent). M0* is the bulk-water intensity of the reference experiment, in which the RF saturation pulse is applied symmetrically on the opposite side of the bulk water signal to correct for non-selective saturation, e.g. direct water saturation.

**Claims**

1. A contrast agent for Magnetic Resonance Imaging (MRI) based on Chemical Exchange-dependent Saturation Transfer (CEST), the agent comprising a non-spherical carrier comprising a lipid bilayer shell enclosing a pool of protons and a paramagnetic chemical shift reagent, wherein the non-spherical carrier is an erythrocyte ghost.

2. A CEST contrast agent according to claim 1, wherein the pool of protons comprises water.

3. A CEST contrast agent according to claim 1 or 2, wherein the paramagnetic shift reagent is a metal complex comprising a metal ion and a ligand that is based on a multidentate chelate ligand.

4. A CEST contrast agent according to claim 3, wherein the metal complex has one coordination site of the metal left open for the coordination of at least one water molecule.

5. A CEST contrast agent according to claim 4, wherein the paramagnetic shift reagent is selected from the group consisting of $[Ln(hpdo3a)(H_2O)]$, $[Ln(dota)(H_2O)]^-$, $[Ln(dotma)(H_2O)]^-$, $[Ln(dotam)(H_2O)]^{3+}$, $[Ln(dtpa)(H_2O)]^{2-}$, derivatives thereof, and mixtures thereof, with Ln being a lanthanide ion.

6. A CEST contrast agent according to any one of the preceding claims, wherein the lipid bilayer wall comprises a paramagnetic material.

7. A CEST contrast agent according to claim 6, wherein the paramagnetic material is selected from the group consisting of at least one of the lanthanide ions $Dy^{3+}$, $Ho^{3+}$, $Er^{3+}$, $Tm^{3+}$, or $Yb^{3+}$ complexed by a multidentate chelating molecule bearing at least one hydrophobic group that comprises at least 6 carbon atoms.

8. A CEST contrast agent according to any one of the preceding claims, wherein the erythrocyte ghosts are of mammalian origin.

**9.** A CEST contrast agent according to claim 8, wherein the erythrocyte ghosts are of human origin.

**10.** A CEST contrast agent according to any one of the preceding claims, comprising a ligand for targeted binding exposed on the outer surface of the carrier.

**11.** A CEST contrast agent according to claim 10, wherein the ligand comprises a hydrophobic tail penetrating into the lipid bilayer shell of the carrier.

**12.** A CEST contrast agent according to claim 10 or 11, wherein the ligand is a disease-specific molecular probe.

**13.** The use of erythrocyte ghosts as a carrier for a Chemical Exchange-dependent Saturation Transfer contrast agent in $^1$H MRI.

**14.** A use according to claim 13, wherein the contrast agent is brought into a person to be subjected to the MRI, and wherein the erythrocyte ghosts originate from said person.

**15.** A method of performing a CEST MRI scan on a person, wherein CEST contrast agents according to any one of claims 1-12 are brought into body fluid of the person and wherein the MRI method includes the application of an RF pulse for which chemically shifted protons in the agent are receptive, so as to saturate the magnetization of said protons, and allowing sufficient time to detect the transfer of said saturation to protons in the outside environment of the contrast agents.

**16.** A method according to claim 15, wherein the contrast agent is obtained on the basis of erythrocyte ghosts originating from the person subjected to the MRI.

**17.** A method of making a CEST contrast agent according to any one of claims 1-12, comprising the steps of (a) providing erythrocytes, (b) subjecting the erythrocyte to hypotonic lysis so as to provide an opening in the erythrocyte membrane, (c) subjecting the opened erythrocyte to one or more washing steps so as to substitute a solution or dispersion of a paramagnetic chemical shift reagent for at least part of the original water-soluble contents of the erythrocyte, and (d) subjecting the resulting paramagnetic chemical shift reagent-loaded erythrocyte ghosts to a closing step under isotonic conditions.

**18.** A method according to claim 17, wherein the erythrocytes are harvested from a person to be subjected to an MRI scan using the resulting CEST contrast agents.

Figure 1

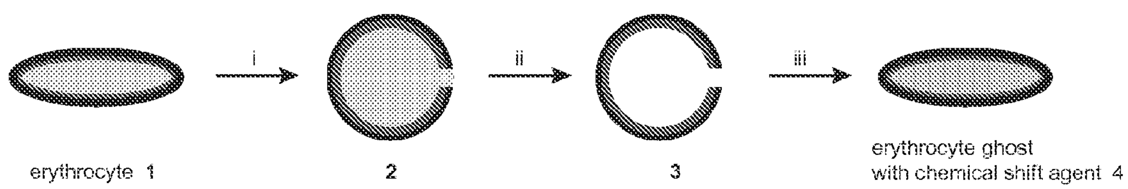


Figure 2

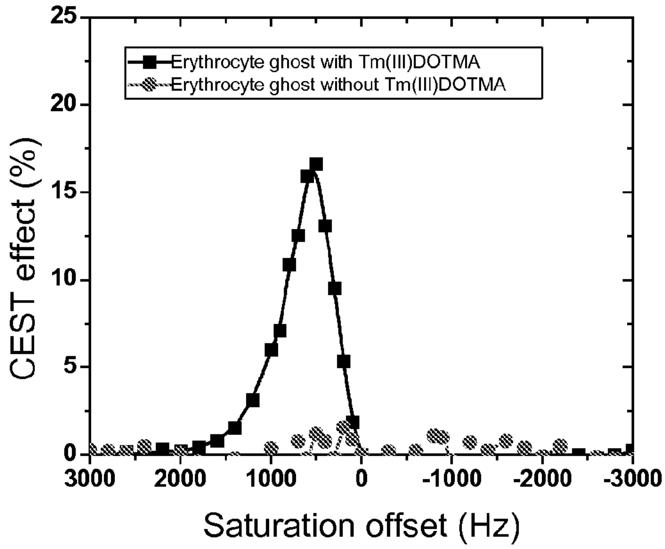

European Patent Office

# EUROPEAN SEARCH REPORT

Application Number
EP 07 12 0180

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | AIME S. ET AL.: "Gd-loaded liposomes as T1, suseptibility, and CEST agents, all in one" JOURNAL OF THE AMERICAN CHEMICAL SOCIETY, vol. 129, no. 9, 7 March 2007 (2007-03-07), pages 2430-2431, XP002470078 * the whole document * ----- | 1-18 | INV. A61K49/18 |
| D,Y | TERRENO E. ET AL.: "From spherical to osmotically shrunken paramagnetic liposomes: an improved generation of LIPOCEST MRI agents with highly shifted water protons" MAGNETIC RESONANCE IMAGING, vol. 46, no. 6, 29 January 2007 (2007-01-29), pages 966-968, XP002470079 * the whole document * ----- | 1-18 | |
| Y | US 2007/243137 A1 (HAINFELD JAMES F [US]) 18 October 2007 (2007-10-18) * page 5, paragraph 48 * * page 7, paragraph 59 * * page 8, paragraph 68 * * page 9, paragraph 70 * ----- | 1-18 | TECHNICAL FIELDS SEARCHED (IPC): A61K |

The present search report has been drawn up for all claims

| Place of search | Date of completion of the search | Examiner |
|---|---|---|
| The Hague | 21 February 2008 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

**ANNEX TO THE EUROPEAN SEARCH REPORT ON EUROPEAN PATENT APPLICATION NO.** EP 07 12 0180

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2008

| Patent document cited in search report | Publication date | Patent family member(s) | Publication date |
|---|---|---|---|
| US 2007243137 A1 | 18-10-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TERRENO,E. et al.** *Angew. Chem. Int. Ed.,* 2007, vol. 46, 966-968
- **MILLAN,C.G. et al.** Drug, enzyme and peptide delivery using erythrocytes as carriers. *Journal of Controlled Release,* 2004, vol. 95, 27-49
- **P. CARAVAN et al.** *Chem. Rev.,* 1999, vol. 99, 2293-2352
- **J. A. PEETERS et al.** *Prog. Nucl. Magn. Reson. Spectr,* 1999, vol. 28, 283-350
- **AIME et al.** *Angew. Chem. Int. Ed.,* 2005, vol. 44, 5513-5515
- **AIME et al.** *JACS,* 2007, vol. 129, 2430-2431